# EUROPEAN PATENT APPLICATION

(11) **EP 2 397 564 A1**
(43) Date of publication of application: **21.12.2011**
(21) Application number: 11169768.6
(22) Date of filing: 14.06.2011
(51) Int. Cl.: C12Q 1/68

(54) **Method of determining lymph node metastasis in a lung cancer, apparatus for determining lymph node metastasis in a lung cancer, and computer program product**

(30) Priority: 15.06.2010 JP 2010136345
(71) Applicant: SYSMEX CORPORATION, Kobe-shi, Hyogo 651-0073 (JP)
(72) Inventor: Hiyama, Kayo, Hyogo 651-0073 (JP); Yoshida, Yuichiro, Hyogo 651-0073 (JP); Kobayashi, Masaki, Hyogo 651-0073 (JP); Nakabayashi, Kadzuki, Hyogo 651-0073 (JP)
(74) Representative: HOFFMANN EITLE

(57) **Abstract**

A method of determining a lymph node metastasis in a lung cancer, comprising: acquiring mRNA expression level of stratifin contained in a measurement sample prepared by using a lymph node tissue of a lung cancer patient who may have a lymph node metastasis; and determining that the lymph node tissue is a lymph node metastasis of the lung cancer when the acquired mRNA expression level of stratifin is overexpressed. An apparatus and a computer program product are also disclosed.

## Description

### FIELD OF INVENTION

The present invention relates to a method of determining lymph node metastasis in a lung cancer, an apparatus for determining lymph node metastasis in a lung cancer, and a computer program product.

### BACKGROUND

In the diagnosis of lung cancer, the presence of lymph node metastasis is useful information to determine the cut extent of lymph node for a functional preservation operation and to determine a chemotherapy after surgery. In many medical institutions, such an examination of the presence of lymph node metastasis is performed by using tissue diagnosis in which a section prepared from a lymph node tissue is observed with a microscope.
However, the tissue diagnosis may not provide a result of accurate diagnosis when a section prepared with a cut surface not containing cancer cells is used or when lymph node metastasis is micrometastasis, even in the presence of the cancer cells in the lymph node tissue.
Further, a result of the tissue diagnosis may vary depending on the skill of a pathologist in charge of the tissue diagnosis.
Thus, a method of examining lung cancer based on the expression of a molecular marker is proposed.

Molecular markers for the lymph node metastasis in lung cancer, such as TACSTD1, CK19, and CEA are described in Liqiang Xi, et. al A Combination of Molecular Markers Accurately Detects Lymph Node Metastasis in Non Small Cell Lung Cancer Patients Clin Cancer Res 2006;12(8) April 15, 2006 p.2484-2491. This discloses a method of examining the presence of lung cancer metastasis to the lymph node using the fact that a possibility of lung cancer metastasis to the lymph node increases with an increase in the expression level of these molecular markers in a lymph node tissue. However, the development of molecular markers and methods is desired to examine the presence of lymph node metastasis in lung cancer with higher accuracy.

On the other hand, it is reported that the expression of a gene called "stratifin (14-3-3σ)" is involved in the control of cancer.
The fact that the mRNA expression of stratifin is controlled in cancer cells in breast cancer as compared with normal cells is disclosed in Anne T. Ferguson, et.al High frequency of hypermethylation at the 14-3-3σ locus leads to gene silencing in breast cancer PNAS May 23, 2000 vol. 97 no.11 6049-6054.
The fact that when the stratifin expression at a protein level in a primary focus of lung cancer is compared with that in a metastatic lymph node tissue, stratifin is down-regulated in the lung cancer metastatic lymph node tissue is reported in Dan-juan Li, et.al "Identificating 14-3-3 sigma as a lymph node metastasis-related protein in humanlung squamous carcinoma"Cancer Letters, 2009 vol.279 65-73.

### SUMMARY

The scope of the present invention is defined solely by the appended claims, and is not affected to any degree by the statements within this summary.
(1) A first aspect of the present invention is a method of determining a lymph node metastasis in a lung cancer, comprising: acquiring mRNA expression level of stratifin contained in a measurement sample prepared by using a lymph node tissue of a lung cancer patient who may have a lymph node metastasis; and determining that the lymph node tissue is a lymph node metastasis of the lung cancer when the acquired mRNA expression level of stratifin is overexpressed. According to the method, the presence of lymph node metastasis in lung cancer can be examined with higher accuracy.
(2) The method of (1), wherein the mRNA expression level of stratifin is acquired by nucleic acid amplification method.
(3) The method of (2), wherein the nucleic acid amplification method is selected from the group consisting of a polymerase-chain-reaction assay, a strand displacement reaction assay, a ligase-chain-reaction assay or a transcriptional amplification method.
(4) The method of (2), wherein the mRNA expression level of stratifin is a value based on the cycle number of nucleic acid amplification reaction by the nucleic acid amplification method.
(5) The method of (4), wherein the determining that the lymph node tissue is a lymph node metastasis of the lung cancer comprises: comparing the cycle number of nucleic acid amplification reaction with a predetermined threshold value; and determining that the lung cancer has spread to the lymph node tissue when the cycle number is smaller than the predetermined threshold value.
(6) The method of (5), wherein the determining that the lymph node tissue is a lymph node metastasis of the lung cancer comprises: determining that the lung cancer has not spread to the lymph node tissue when the cycle number is higher than the predetermined threshold value.
(7) The method of (2), wherein the mRNA expression level of stratifin is a quantitative value of mRNA of stratifin which is calculated by the nucleic acid amplification method.
(8) The method of (7), wherein the determining that the lymph node tissue is a lymph node metastasis of the lung cancer further comprises: comparing the calculated quantitative value of mRNA of stratifin with a predetermined threshold value, and determining that the lung cancer has spread to the lymph node tissue when the quantitative value is higher than the predetermined threshold value.
(9) The method of (8), wherein the determining that the lymph node tissue is a lymph node metastasis of the lung cancer further comprises: determining that the lung cancer has not spread to the lymph node tissue when the quantitative value is smaller than the predetermined threshold value.
(10) The method of any one of (1) to (9), wherein the lymph node tissue is a tissue containing a lymph node belonging to a lung.
(11) The method of (10), wherein the lymph node belonging to the lung is any one of mediastinal lymph nodes such as upper longitudinal lymph nodes, paratracheal lymph nodes, pretracheal lymph nodes, anterior mediastinal lymph nodes, retrotracheal lymph nodes, tracheobronchial lymph nodes, subaortic lymph nodes, paraaortic lymph nodes, tracheal-bifurcation lymph nodes, juxtaesophageal lymph nodes and pulmonary ligament lymph nodes; hilar lymph nodes such as primary peribronchial lymph nodes, lobar bronchi lymph nodes and lobar peribronchial lymph nodes; segmental peribronchial lymph nodes, and subsegmental bronchus lymph nodes.
(12) The method of any one of (1) to (11), wherein the measurement sample prepared using the lymph node tissue is a sample obtained by homogenizing a tissue which may have a metastasis in lung cancer in the pretreatment liquid.
(13) The method of (12), wherein a pH of the pretreatment liquid is from 2.5 to 5.0.
(14) A second aspect of the present invention is an apparatus for determining lymph node metastasis in lung cancer, comprising: a measurement unit which acquires mRNA expression level of stratifin contained in a measurement sample prepared by using a lymph node tissue which may have a metastasis in lung cancer; a determination unit which determines that lung cancer has spread to the lymph node tissue when the mRNA expression of stratifin obtained by the measurement unit is overexpressed; and an output unit which outputs the determination result obtained by the determination unit. Such a configuration allows the presence of lymph node metastasis in lung cancer to be examined with higher accuracy.
(15) A third aspect of the present invention is a computer program product, comprising: a computer readable medium; and instructions, on the computer readable medium, adapted to enable a general purpose computer to perform operations, comprising: receiving mRNA expression level of stratifin contained in a measurement sample prepared by using a lymph node tissue which may have a metastasis in lung cancer; determining that lung cancer has spread to the lymph node tissue when the mRNA expression of stratifin obtained in the receiving step is overexpressed; and outputting the determination result obtained by the determining step. Such a configuration allows the presence of lymph node metastasis in lung cancer to be examined with higher accuracy.

### BRIEF DESCRIPTION OF THE DRAWINGS

Fig. 1 shows an embodiment of a determining device which executes determination of lymph node metastasis in lung cancer;
Fig. 2 is a flow chart showing an example of determination of lymph node metastasis in lung cancer; and
Fig. 3 is a graph showing the presence of lymph node metastasis and a relationship between the type of marker and the number of PCR cycles in Experimental example 1.

### DETAILED DESCRIPTION OF THE EMBODIMENT

### [1. Examination method of lymph node metastasis in lung cancer]

An examination method of lymph node metastasis in lung cancer of the present invention (hereinafter simply referred to as an "examination method") includes acquiring mRNA expression level of stratifin contained in a measurement sample prepared by using a lymph node tissue from a lung cancer patient which may have a metastasis in lung cancer and determining that lung cancer has spread to the lymph node tissue when the acquired RNA expression of stratifin is overexpressed.

Stratifin is a protein which is considered to have a function to stop the cell cycle at G2/M phase and is also referred to as 14-3-3-σ. The base sequence of the mRNA of stratifin is available as the GenBank accession number: NM_006142 from the GenBank database provided by National Center for Biotechnology Information. The GenBank accession number indicates the most recent release number as of Mar. 14, 2010.

### [1-1. Acquisition of expression level]

The examination method of the present invention includes acquiring mRNA expression level of stratifin contained in a measurement sample prepared by using a lymph node tissue from a lung cancer patient which may have a metastasis in lung cancer.

The measurement sample may be preferably a sample suitable for measuring the expression level of mRNA of stratifin. For example, the measurement sample may be obtained by mixing cells contained in the lymph node tissue with the suitable pretreatment liquid, subjecting cells in the obtained mixture to a chemical process and/or a physical process, and extracting RNA from the cells contained in the lymph node tissue using a commercially available RNA extraction kit. In the process of preparing the measurement sample, the measurement sample is prepared simply in a short time, and thus it is preferably performed by a method using the pretreatment liquid.

The lymph node tissue may be a tissue including a lymph node belonging to a lung. Examples of the lymph node include mediastinal lymph nodes such as upper longitudinal lymph nodes, paratracheal lymph nodes, pretracheal lymph nodes, anterior mediastinal lymph nodes, retrotracheal lymph nodes, tracheobronchial lymph nodes, subaortic lymph nodes, paraaortic lymph nodes, tracheal-bifurcation lymph nodes, juxtaesophageal lymph nodes and pulmonary ligament lymph nodes; hilar lymph nodes such as primary peribronchial lymph nodes, lobar bronchi lymph nodes and lobar peribronchial lymph nodes; intrapulmonary lymph nodes such as segmental peribronchial lymph nodes and subsegmental bronchus lymph nodes. These lymph nodes may be suitably selected depending on the condition of patients being subjected to the examination method according to the present embodiment.

The pretreatment liquid may be a solution which solubilizes mRNA in cells contained in a lymph node tissue. From the viewpoint of controlling degradation of RNA, the pretreatment liquid has preferably an acid pH and the range is preferably from pH 2.5 to pH 5.0, more preferably from pH 3.0 to pH 4. 0. As the pretreatment liquid, for example, a solution including a buffer is listed. As the buffer, for example, a glycine chloride buffer is listed. The concentration of the buffer in the pretreatment liquid may be suitably set within a range which keeps the pH of the pretreatment liquid acidic.

It is preferable that the pretreatment liquid further contains a surfactant from the viewpoint to improve the extraction efficiency of mRNA from cells contained in the lymph node tissue. The surfactant is not particularly limited as long as it is capable of damaging cell membranes and nuclear membranes of cells contained in the lymph node tissue and allowing nucleic acids in the cells to be easily extracted. Examples of the surfactant include nonionic surfactants. Among the nonionic surfactants, a polyoxyethylene-based nonionic surfactant is preferred and a polyoxyethylene-based nonionic surfactant represented by Formula (I) is more preferred from the viewpoint of improving the extraction efficiency of mRNA from cells contained in the lymph node tissue:

R-¹-R²-(CH₂CH₂O)-H (I)

(wherein R¹ represents an alkyl group having 10 to 22 carbon atoms, an alkenyl group having 10 to 22 carbon atoms, an alkynyl group having 10 to 22 carbon atoms or an isooctyl group having 10 to 22 carbon atoms, R² represents an oxygen atom or a phenyleneoxy group, and n represents an integer of 8 to 120). The polyoxyethylene-based nonionic surfactant is not particularly limited. Examples thereof include polyoxyethylene lauryl ether, polyoxyethylene cetyl ether, polyoxyethylene oleyl ether, polyoxyethylene myristyl ether, polyoxyethylene stearyl ether, polyoxyethylene nonylphenyl ether, and polyoxyethylene isooctyl phenyl ether. As the polyoxyethylene-based nonionic surfactant, polyoxyethylene lauryl ether (addition mole number of an oxyethylene group: 23) (trade name: Brij35, manufactured by Sigma Aldrich) or the like may be used. The concentration of the surfactant in the pretreatment liquid is preferably from 0.1 to 6% by volume, more preferably from 1 to 5% by volume from the viewpoint of sufficiently extracting mRNA from cells contained in the lymph node tissue.

When acquiring mRNA expression level of stratifin by a method of amplifying nucleic acid to be described later, it is preferable that the pretreatment liquid contains dimethyl sulfoxide (hereinafter referred to as "DMSO"). This allows a decrease in the activity of a nucleic acid synthesis enzyme which is used for the method of amplifying nucleic acid to be controlled. Besides, even if a substance (inhibitor) which inhibits the reaction of the nucleic acid synthesis enzyme is contained in the lymph node tissue, the influence of the inhibitor may be effectively reduced. The concentration of DMSO in the pretreatment liquid is preferably from 1 to 50% by volume, more preferably 5 to 30% by volume, still more preferably from 10 to 25% by volume from the viewpoint of sufficiently exhibiting the effect.

The amount of the pretreatment liquid which is used for the preparation of the measurement sample varies depending on the type of the pretreatment liquid, and thus it may be suitably set according to the type of the pretreatment liquid. Usually, the amount of the pretreatment liquid which is used for the preparation of the measurement sample is from 0.0001 to 0.0005 mL per 1 mg of lymph node tissue.
A mixing process of cells contained in the lymph node tissue and the pretreatment liquid is performed by, for example, stirring the cells contained in the lymph node tissue and the pretreatment liquid at room temperature.

Examples of the chemical process include a process of solubilizing cells with a surfactant and a process of separating RNA with an organic solvent such as chloroform. Examples of the physical process include a fracturing process using a homogenizer and a freezing and thawing process. Products obtained by the chemical process and/or the physical process may be purified by a refining method such as centrifugation, filtering or column chromatography, if necessary.

A process of acquiring mRNA expression level of stratifin may be performed by known methods such as the method of amplifying nucleic acid and DNA microarray hybridization method using DNA microarray in which nucleic acids corresponding to mRNA of stratifin are arranged.

In the present specification, the "mRNA expression level of stratifin" in the case of the method of amplifying nucleic acid is not particularly limited as long as it is data about the mRNA expression level of stratifin. The term "mRNA expression level of stratifin" means, for example, an optical measurement value (e.g. the fluorescence intensity, turbidity, and absorbance) based on the amplified mRNA, the cycle number or time when the optical measurement value reaches a predetermined standard value, the cycle number or time when the variation of the optical measurement value in nucleic acid amplification reaction reaches a predetermined standard value, and the quantitative value (expression level) of mRNA calculated from the optical measurement value, the cycle number, and the calibration curve. In the present specification, the term "data about the mRNA expression level of stratifin" in the case of the method using DNA chip means, for example, the fluorescence intensity based on mRNA of stratifin hybridized with the nucleic acids on a DNA chip, the quantitative value of mRNA (expression level) calculated from the fluorescence intensity, and the calibration curve. The term "variation of the optical measurement value in nucleic acid amplification reaction" means the variation of optical measurement value before and after one cycle of nucleic acid amplification reaction.
Here, the predetermined standard value may be suitably set according to the type of the data. For example, when the data is the cycle number, the variation of fluorescence intensity when the nucleic acid amplification reaction shows a logarithmic growth may be set as a standard value.

The mRNA expression level of stratifin may be acquired by the method of amplifying nucleic acid because of easy acquisition of the data regarding the mRNA expression level of stratifin.

The method of amplifying nucleic acid may be performed by maintaining a reaction solution containing, for example, the measurement sample, a primer pair (or a primer set) for amplifying mRNA of stratifin, a part of the mRNA, nucleic acid corresponding to the mRNA (e.g. cDNA), and the nucleic acid synthesis enzyme under a suitable reaction condition.

Examples of the method of amplifying nucleic acid include a polymerase-chain-reaction assay, a strand displacement activity assay, a ligase-chain-reaction assay, and a transcriptional amplification method. As the polymerase-chain-reaction assay, for example, a quantitative RT-PCR (Reverse Transcription PCR) assay is listed. As the strand displacement activity assay, for example, a quantitative RT-LAMP (Reverse Transcription LAMP) assay (refer to, for example, U.S. Patent No. 6410278) is listed. As the transcriptional amplification method, for example, a TAS assay is listed. Among these methods, the quantitative RT-PCR assay and the quantitative RT-LAMP assay are preferred from the viewpoint of easy acquisition of the data.

Examples of the quantitative RT-PCR assay include a TaqMan method ("TaqMan" is a registered trademark of Roche Molecular Systems Inc.) and an intercalator method using an intercalator (e.g. trade name: SYBR Green, manufactured by Molecular Probe Inc.). In the quantitative RT-PCR assay, the optical state of the reaction solution changes with the amplification of nucleic acid, and thus rapid and simple acquisition of the expression level is achieved by real-time measurement of the optical measurement value of the reaction solution.

In the quantitative RT-LAMP assay, magnesium pyrophosphate which is insoluble in a liquid component contained in the reaction solution is produced by the amplification of cDNA corresponding to mRNA of stratifin. Therefore, in the quantitative RT-LAMP assay, the data about the mRNA expression level of stratifin may be calculated based on the turbidity of the reaction solution or the time until the absorbance reaches a predetermined standard value.

The primer included in the primer pair (or the primer set) is designed according to the type of the method of amplifying nucleic acid based on a base sequence of mRNA of stratifin. When the method of amplifying nucleic acid is the quantitative RT-PCR assay, the primer pair (or the primer set) is not particularly limited. For example, a primer pair including a forward primer consisting of a base sequence of SEQ ID NO: 1 (5' -GCAGGCCGAACGCTATGA-3') and a reverse primer consisting of a base sequence of SEQ ID NO: 2 (5'-GCAGGTTTCGCTCTTCGCA-3') is listed. The primer included in the primer pair (or the primer set) is preferably labeled with a labeling substance from the viewpoint of easy measurement of the optical measurement value of the reaction solution. The labeling substance is not particularly limited and examples thereof include radioisotopes, fluorescent substances, ligands, and pigments.

The nucleic acid synthesis enzyme may be selected according to the type of the method of amplifying nucleic acid. In the present specification, the nucleic acid synthesis enzyme means, for example, RNA-dependent DNA polymerase (hereinafter referred to as "reverse transcriptase"), DNA dependent DNA polymerase (hereinafter referred to as "DNA polymerase,"), strand-displacement-type DNA polymerase, ligase, or the like. When the method of amplifying nucleic acid is the quantitative RT-PCR assay, reverse transcriptase and DNA polymerase are used as the nucleic acid synthesis enzyme. Examples of the reverse transcriptase include AMV reverse transcriptase and M-MLV reverse transcriptase. Examples of the DNA polymerase include Taq DNA polymerase, T4 DNA polymerase, and Bst DNA polymerase. In the quantitative RT-PCR assay, an enzyme having both reverse transcriptase activity and DNA synthesis activity may be used as the nucleic acid synthesis enzyme.

Since the reaction condition for the method of amplifying nucleic acid varies depending on the type of the method of amplifying nucleic acid and the type of the primer pair to be used, it is not necessarily determined. The reaction condition may be suitably set according to the type of the method of amplifying nucleic acid and the type of the primer pair to be used with reference to the method described in [Sambrook et al., Molecular Cloning Laboratory Manual, 2nd ed. (1989)].

### [1-2. Determination of metastasis in lung cancer]

Subsequently, it is determined that lung cancer has spread to the lymph node tissue when the acquired mRNA expression of stratifin is overexpressed.

Comparison of the data about the expression level with the threshold value according to the type of the data allows for determination if the mRNA expression of stratifin is overexpressed. Here, the wording "mRNA expression of stratifin is overexpressed" means that, for example, the data about the expression level indicates that the mRNA expression level of stratifin is higher than the expression level corresponding to the threshold value. For example, the case where the data is the cycle number when the variation of fluorescence intensity in the nucleic acid amplification reaction reaches a predetermined standard value shows that the mRNA expression of stratifin is overexpressed when "the cycle number is less than the threshold value". In this case, it is determined that lung cancer has spread to the lymph node tissue. The case where "the cycle number is greater than the threshold value" shows that the mRNA expression of stratifin is not overexpressed. In this case, it is determined that lung cancer has not spread to the lymph node tissue.
The case where the data is the quantitative value (expression level) of mRNA shows that the mRNA expression of stratifin is overexpressed when "the quantitative value (expression level) is greater than the threshold value of mRNA". In this case, it is determined that lung cancer has spread to the lymph node tissue. The case where "the quantitative value of mRNA (expression level) is less than the threshold value" shows that the mRNA expression of stratifin is not overexpressed. In this case, it is determined that lung cancer has not spread to the lymph node tissue.

The threshold value may be set by various methods according to the type of the data about the expression level. That is, the threshold value may be experientially set to a value having a predetermined expression level which may be classified into two categories: a lymph node group in which lung cancer metastasis is observed and a lymph node group in which lung cancer metastasis is not observed. More specifically, when the data about the expression level is the cycle number, the cycle number regarding a plurality of lymph nodes in which lung cancer metastasis is histologically observed and a plurality of lymph nodes in which lung cancer metastasis is not histologically observed may be acquired, and the cycle number which classifies into two groups: a lymph node group in which lymph node metastasis is observed and a lymph node group in which lymph node metastasis is not observed may be set as a threshold value.

### [2 . Device for determining lymph node metastasis in lung cancer]

Subsequently, as an example of a device suitable for performing the examination method according to an embodiment of the present invention, the device for determining lymph node metastasis in lung cancer according to an embodiment of the present invention (hereinafter also referred to as a "determining device") will be described in detail with reference to the accompanying drawing.
The determining device according to the present embodiment performs determination by comparing the cycle number with a threshold value of the cycle number using the cycle number when the variation of fluorescence intensity reaches the predetermined standard value as the data about the expression level of the mRNA.

### [2-1. Configuration of determining device]

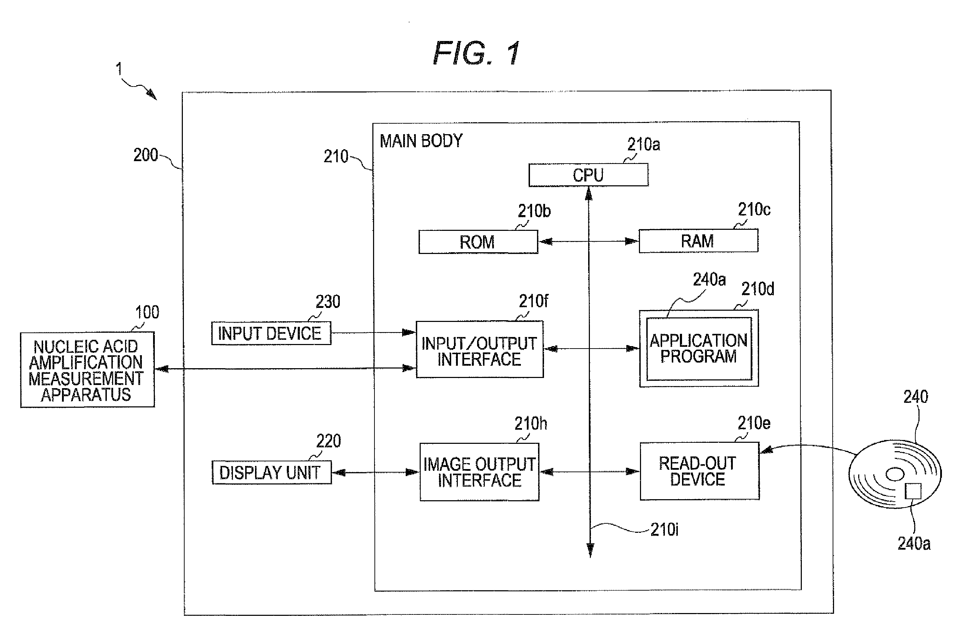

Fig. 1 is a block diagram showing the entire configuration of a determining device 1 according to an embodiment of the present invention.
The determining device 1 according to the present embodiment is configured to have a nucleic acid amplification measurement apparatus 100 and an information processing apparatus 200 connected to the nucleic acid amplification measurement apparatus 100.
The nucleic acid amplification measurement apparatus (measurement unit) 100 includes a PCR device capable of performing a quantitative RT-PCR assay. The nucleic acid amplification measurement apparatus 100 amplifies mRNA of stratifin and measures the fluorescence intensity based on the amplified mRNA and the cycle number in quantitative RT-PCR assay.
The information processing apparatus (computer) 200 is connected to the nucleic acid amplification measurement apparatus 100.
The information processing apparatus 200 controls the operation of the nucleic acid amplification measurement apparatus 100 and determines whether the lymph node metastasis is positive using the data of the fluorescence intensity and the data of the cycle number which have been measured by the nucleic acid amplification measurement apparatus 100.

The information processing apparatus 200 includes an information processing apparatus main body (determining unit) 210, an input device 230 which inputs necessary data to the information processing apparatus main body 210, and a display unit (outputting unit) 220 which displays input/output data. An external recording medium 240 is included in the information processing apparatus 200, if necessary.

The information processing apparatus main body 210 includes a CPU 210a, a ROM210b, a RAM210c, a hard disk 210d, a read-out device 210e, an input/output interface 210f, an image output interface 210h, and a bus 210i.
In the information processing apparatus main body 210, the CPU 210a, the ROM210b, the RAM210c, the hard disk 210d, the read-out device 210e, the input/output interface 210f, and the image output interface 210h are respectively connected by the bus 210i so as to transmit and receive data.

The CPU 210a can execute computer programs stored in the ROM 210b and the computer programs loaded in the RAM 210c. A computer program which is executed by the CPU 210a and data for the computer program which is executed by the CPU 210a are recorded on the ROM 210b. The RAM 210c is used to read out the computer programs recorded on the ROM 210b and the hard disc 210d. In executing the computer program, the RAM 210c is used as a work region of the CPU 210a.
The hard disc 210d is installed with various computer programs to be executed by the CPU 210a such as operating system (OS) and application program, as well as data used in executing the computer program.
The program installed on the hard disk 210d includes an application program 240a which realizes the method of examining lymph node metastasis in lung cancer and a program which controls the nucleic acid amplification measurement apparatus 100. As data to be used for executing the program which realizes the method of examining lymph node metastasis in lung cancer, threshold values or the like are stored in the hard disk 210d.

The read-out device 210e is configured by flexible disc drive, CD-ROM drive, DVD-ROM drive, and the like, and is able to read out computer programs and data recorded on an external recording medium 240. The application program 240a may be recorded on the external recording medium 240, the ROM 210b or the RAM 210c formed in the information processing apparatus main body 210.
The configuration in which the CPU 210a reads out the application program 240a from the external recording medium 240 and the application program 240a is installed on the hard disk 210d may be employed.

Operating system providing graphical user interface environment such as Windows (registered trademark) manufactured and sold by US Microsoft Co. is installed in the hard disc 210d. In the following description, the application program 240a according to the above determination is assumed to be operating on the operating system.

The input/output interface 210f includes a serial interface such as USB, IEEE1394, and RS-232C; a parallel interface such as SCSI, IDE, and IEEE1284; and an analog interface such as D/A converter and A/D converter. The input/output interface 210f is connected to the input device 230 such as a keyboard and a mouse. The nucleic acid amplification measurement apparatus 100 is connected to the input/output interface 210f. Thus, the information processing apparatus main body 210 can transmit and receive data with the nucleic acid amplification measurement apparatus 100 via the input/output interface 210f.

The image output interface 210h is connected to the display 220 configured by LCD, CRT, or the like, and outputs an image signal corresponding to the image data provided from the CPU 210a to the display 220. The display 220 displays the image (screen) according to the input image signal. The display unit 220 displays the determination result obtained from the CPU 210a to be described later.

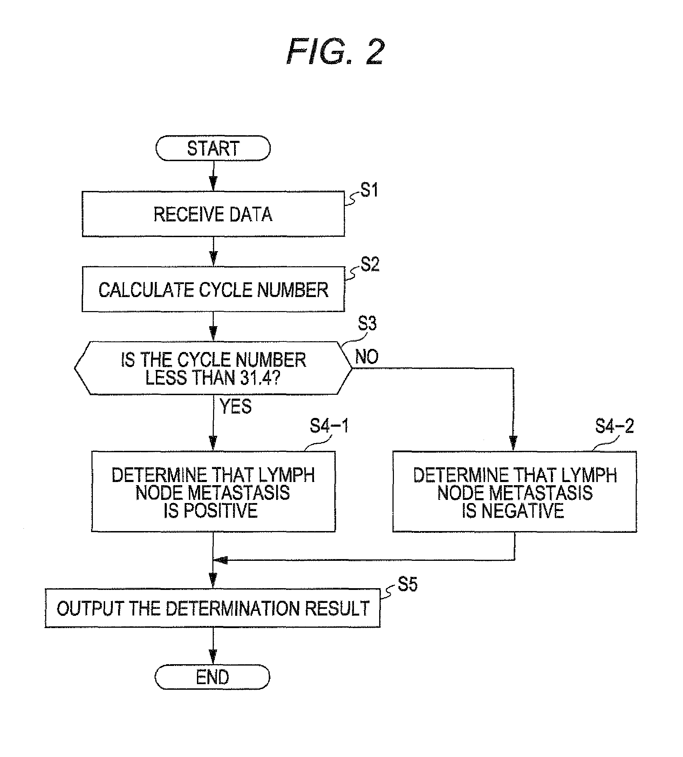

Here, an example of an operation flow of the application program 240a which is performed by the determining device 1 will be described using Fig. 2.

First, the CPU 210a receives the data of the variation of fluorescence intensity in the nucleic acid amplification reaction and the data of the cycle number from the nucleic acid amplification measurement apparatus 100 via the input/output interface 210f (step S1).

In step S2, the CPU 210a calculates the cycle number (cycle number A) when the variation of fluorescence intensity in the nucleic acid amplification reaction reaches a standard value of 0. 08 based on the data of the variation of fluorescence intensity in the nucleic acid amplification reaction and the data of the cycle number.

In step S3, the CPU 210a compares the data of a threshold value (31.4 cycles) of the cycle number which has been previously stored as the data of the application program 240a in the hard disk 210d with the data of the cycle number A. In step S3, the cycle number A is compared with the threshold value (31.4 cycles) of the cycle number to determine whether it is smaller than the threshold value.
When the cycle number A is smaller than 31.4 cycles (Yes), the CPU 210a proceeds the process to step S4-1. On the other hand, when the cycle number A is not smaller than 31.4 cycles (No), the CPU 210a proceeds the process to step S4-2.

In step S4-1, the CPU 210a determines that lung cancer has spread to the lymph node tissue (positive for lymph node metastasis). In step S4-2, the CPU 210a determines that lung cancer has not spread to the lymph node tissue (negative for lymph node metastasis).

Thereafter, the CPU 110a stores the determination result in the RAM210c and outputs them to the display unit 220 via the image output interface 210h in step S5.

In the present embodiment, the data of the variation of fluorescence intensity in the nucleic acid amplification reaction and the data of the cycle number are configured to be acquired from the nucleic acid amplification measurement apparatus 100 via the input/output interface 210f, however, the present invention is not limited thereto. It may be configured that the data of the variation of fluorescence intensity in the nucleic acid amplification reaction and the data of the cycle number are input by the input device 230, and the CPU 210a acquires the cycle number (cycle number A) when the variation of fluorescence intensity in the nucleic acid amplification reaction reaches the standard value by calculating from the input values. The term "variation of fluorescence intensity in the nucleic acid amplification reaction" means, for example, the variation of fluorescence intensity before and after one cycle in reaction of nucleic acid amplification reaction.
In the present embodiment, it is configured that the CPU 210a acquires the cycle number (cycle number A) when the variation of fluorescence intensity in the nucleic acid amplification reaction reaches the standard value by calculating from the data of the variation of fluorescence intensity in the nucleic acid amplification reaction and the data of the cycle number, however the present invention is not limited thereto. The data may be acquired by inputting the cycle number (cycle number A) when the variation of fluorescence intensity in the nucleic acid amplification reaction reaches the standard value by the input device 230.

In the present embodiment, the CPU 210a calculates the cycle number when the variation of fluorescence intensity in the nucleic acid amplification reaction reaches the standard value based on the data of the variation of fluorescence intensity in the nucleic acid amplification reaction and the data of the cycle number, however the present invention is not limited thereto. The quantitative value of mRNA of stratifin may be calculated from the fluorescence intensity, cycle number, and calibration curve. In this case, the process may be proceeded to step S4-1 when the quantitative value is higher than a predetermined threshold value in step S3, and the process may be proceeded to step S4-2 when the quantitative value is smaller than the predetermined threshold value.

### Examples

The present invention will be described in detail below with reference to examples, however the present invention is not limited thereto.

### (Preparation example 1)

Measurement samples were prepared in the following manner using 15 lymph nodes in which lung cancer metastasis was histologically observed [positive specimens (lymph nodes positive for metastasis) 1 to 15] and 20 lymph nodes in which lung cancer metastasis was not histologically observed [negative specimens (lymph nodes negative for metastasis) 1 to 20] . The site and name of lymph nodes of the positive specimens 1 to 15 are shown in Table 1. The site and name of lymph nodes of the negative specimens 1 to 20 are shown in Table 2.

**[Table 1]**

| | Lymph node Number | Lymph node |
|---|---|---|
| 1 | #2 | Paratracheal lymph nodes |
| 2 | #2 | Paratracheal lymph nodes |
| 3 | #3 | Anterior mediastinal lymph nodes |
| 4 | #3a | Anterior mediastinal lymph nodes |
| 5 | #3, #4 | Pretracheal lymph nodes |
| | | Tracheobronchial lymph nodes |
| 6 | #4 | Tracheobronchial lymph nodes |
| 7 | #5 | Subaortic lymph nodes |
| 8 | #6 | Paraaortic lymph nodes |
| 9 | #10 | Primary peribronchial lymph nodes |
| 10 | #10 | Primary peribronchial lymph nodes |
| 11 | #11 | Lobar bronchi lymph nodes |
| 12 | #11i | Lobar bronchi lymph nodes |
| 13 | #11i | Lobar bronchi lymph nodes |
| 14 | #12u | Lobar peribronchial lymph nodes |
| 15 | #12u | Lobar peribronchial lymph nodes |

**[Table 2]**

| | Lymph node Number | Lymph node |
|---|---|---|
| 1 | #2 | Paratracheal lymph nodes |
| 2 | #2, #3 | Paratracheal lymph nodes |
| | | Anterior mediastinal lymph nodes |
| 3 | #3 | Anterior mediastinal lymph nodes |
| 4 | #3 | Anterior mediastinal lymph nodes |
| 5 | #3, #4 | Pretracheal lymph nodes |
| | | Tracheobronchial lymph nodes |
| 6 | #4 | Tracheobronchial lymph nodes |
| 7 | #4 | Tracheobronchial lymph nodes |
| 8 | #4 | Tracheobronchial lymph nodes |
| 9 | #4 | Tracheobronchial lymph nodes |
| 10 | #5 | Subaortic lymph nodes |
| 11 | #5 | Subaortic lymph nodes |
| 12 | #6 | Paraaortic lymph nodes |
| 13 | #6 | Paraaortic lymph nodes |
| 14 | #6 | Paraaortic lymph nodes |
| 15 | #7 | Tracheal-bifurcation lymph nodes |
| 16 | #10 | Primary peribronchial lymph nodes |
| 17 | #11 | Lobar bronchi lymph nodes |
| 18 | #11i | Lobar bronchi lymph nodes |
| 19 | #12i | Lobar peribronchial lymph nodes |
| 20 | #12u | Lobar peribronchial lymph nodes |

First, 4 mL of a solubilized liquid [composition: 200 mM glycine chloride (pH 3.4), 5% by volume of polyoxyethylene lauryl ether (addition mole number of an oxyethylene group: 23) (trade name: Brij35, manufactured by Sigma Aldrich), 20% by volume of dimethyl sulfoxide, and 0.05% by volume of an antifoaming agent [trade name: KS-538, manufactured by Shin-Etsu Chemical Co., Ltd.]] was added to the lymph nodes (about 30 to 370 mg/ (piece)). Subsequently, the lymph node was homogenized with a blender. The obtained homogenate was centrifuged at 10000xg for 1 minute at room temperature to obtain a supernatant. Then, RNAs were extracted from 400 µL of the supernatant using a kit for RNA extraction/purification (trade name: RNeasy Mini kit, manufactured by QIAGEN, catalog number 74014) and purified them to obtain 60 µL of an RNA solution. The absorbance of the obtained RNA solutions at a wavelength of 260 nm was measured. The obtained RNA solutions were used as measurement samples.

### (Experimental example 1)

Real-time RT-PCR assay was performed by a real-time PCR device (trade name: ABI Prism 7500, manufactured by Applied Biosystems) using the measurement samples obtained in Preparation example 1, a primer pair for stratifin, and a quantitative RT-PCR kit (trade name: Quanti Tect SYBR Green RT-PCR kit, manufactured by QIAGEN, catalog number 204245). As for the measurement samples, the cycle number of PCR when the variation of fluorescence intensity based on amplification products reached the standard value was determined. The obtained cycle number of PCR was used as an index of the expression level of mRNA of stratifin in the measurement samples (Example 1).

The operation was performed in the same manner as described above except that a primer pair for CEA (Comparative example 1) or a primer pair for TACSTD1 (Comparative example 2) was used in place of the primer pair for stratifin. As for the measurement samples, the cycle number of PCR when the variation of fluorescence intensity based on amplification products reached the standard value was determined. CEA and TACSTD1 are known molecular markers for lymph node metastasis in lung cancer.

As a control, the operation was performed in the same manner as described above except that a primer pair for β-actin was used in place of the primer pair for stratifin. As for the measurement samples, the cycle number of PCR when the variation of fluorescence intensity based on amplification products reached the standard value was determined.

As for Example 1 and Comparative examples 1 and 2, a difference between a minimum value of the cycle number of PCR when using the measurement sample of the positive specimen and a maximum value of the cycle number of PCR when using the measurement sample of the negative specimen (a difference in the cycle number of PCR between the positive specimen and the negative specimen) was calculated.

The standard value of the variation of fluorescence intensity when amplifying mRNA of stratifin is 0.08, the standard value when amplifying mRNA of CEA is 0.02, and the standard value when amplifying mRNA of TACSTD1 is 0.02. These standard values indicate the variation of fluorescence intensity when nucleic acid amplification reactions of mRNA of stratifin, mRNA of CEA, and mRNA of TACSTD1 are in a logarithmic phase. These standard values are set so as to be equal to the almost same value when converted to the variation of fluorescence intensity per unit chain length.

The primer pair used for the real-time RT-PCR assay is shown in Table 3 and the composition of the reaction mixture is shown in Table 4.

**[Table 3]**

| | Forward primer | SEQ ID NO: | Reverse primer | SEQ ID NO: |
|---|---|---|---|---|
| Primer pair for stratifin | 5' -GCAGGCCGAACGCTATGA-3' | 1 | 5'-GCAGGTTTCGCTCTTCGCA-3' | 2 |
| Primer pair for CEA | 5' -AGACAATCACAGTCTCTGCGGA-3' | 3 | 5'-ATCCTTGTCCTCCACGGGTT-3' | 4 |
| Primer pair for TACSTD1 | 5' -GTTCGGGCTTCTGCTTGC-3' | 5 | 5'-GTAGTTTTCACAGACACATTCTTC CTG-3' | 6 |
| Primer pair for β-actin | 5' -CCACACTGTGCCCATCTACG-3' | 7 | 5'-AGGATCTTCATGAGGTAGTCAGTC AG-3' | 8 |

**[Table 4]**

| | Volume (µL) |
|---|---|
| RNase-free water | 10.95 |
| 2xQuantiTect SYBR Green RT-PCR Master Mix^{*1} (trade name) | 12.50 |
| 100 nM forward primer solution | 0.15 (Final concentration 600µM) |
| 100 nM reverse primer solution | 0.15 (Final concentration 600µM) |
| Quanti Tect RT Mix^{*2} | 0.25 |
| Measurement sample | 1.00 |
| Total | 25.00 |

| | |
|---|---|
| Superior symbols *1 and *2 represent reagents attached to a quantitative RT-PCR kit. | |

Thermal profiles in the real-time RT-PCR assay show that a cycle of keeping the measurement sample warm at 50°C for 30 minutes, keeping it warm at 95°C for 10 minutes, denaturalizing it at 94°C for 15 seconds, annealing it at 53°C for 30 seconds, and elongating it at 72°C for 30 seconds was carried out 40 times.

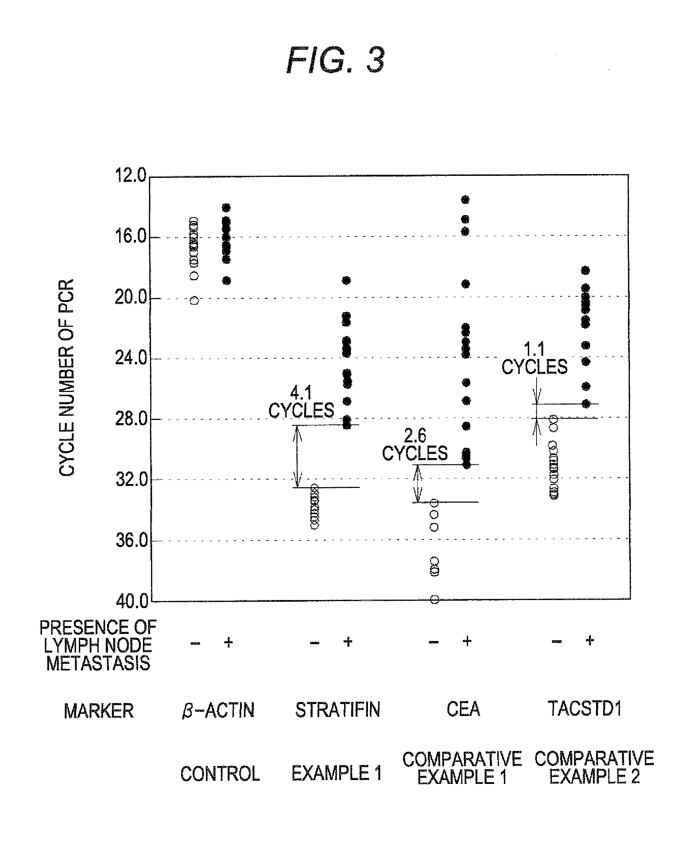

In Experimental example 1, the presence of lymph node metastasis and a relationship between the type of marker and the cycle number of PCR are shown in Fig. 3. In Fig. 3, "the cycle number of PCR" represents the cycle number when the variation of fluorescence intensity based on amplification products reach each of the standard values in the real-time RT-PCR assay using the measurement sample of the positive specimen or the measurement sample of the negative specimen. In Fig. 3, the "+" mark indicates the cycle number of PCR when the measurement sample of the positive specimen is used and the "-" mark indicates the cycle number of PCR when the measurement sample of the negative specimen is used.

As is apparent from the results shown in Fig. 3, a difference in the cycle number of PCR between the positive specimen and the negative specimen when stratifin is used as a molecular marker (example 1) is larger than that in the cycle number of PCR between the positive specimen and the negative specimen when CEA (Comparative example 1) or TACSTD1 (Comparative example 2) is used. As is apparent from the results, a difference in the mRNA expression level of stratifin between the positive specimen and the negative specimen is larger than that in the expression level of mRNA of CEA or TACSTD1 between the positive specimen and the negative specimen. Since the difference in the mRNA expression level of stratifin between the positive specimen and the negative specimen is large, it is considered that a threshold value for distinguishing the positive specimen in which lung cancer has spread to lymph node from the negative specimen is easily set. Therefore, according to the case of stratifin, the positive specimen is clearly distinguished from the negative specimen, as compared with the case of CEA or TACSTD1. This suggests that the presence of lung cancer metastasis to the lymph node tissue may be examined with high accuracy.

As is apparent from the results shown in Fig. 3, a variation range of the cycle number of PCR between the positive specimens and a variation range of the cycle number of PCR between the negative specimens when stratifin is used are smaller respectively than that when CEA is used. As is apparent from the results, mRNA expression variation of stratifin between the positive specimens and mRNA expression variation of stratifin between the negative specimens are smaller respectively than that of CEA between the positive specimens and that of CEA between the negative specimens. Thus, when stratifin is used as a molecular marker in the process of examining lung cancer metastasis to the lymph node tissue, it is considered that, based on information regarding the mRNA expression level of stratifin in a specimen, the specimen may be easily classified into the positive specimen or the negative specimen.

The above-described results suggest that, according to the case of stratifin, lymph node metastasis in lung cancer may be examined with high accuracy as compared with the cases of known molecular markers such as CEA and TACSTD1.

## Claims

1. A method of determining a lymph node metastasis in a lung cancer, comprising:
acquiring mRNA expression level of stratifin contained in a measurement sample prepared by using a lymph node tissue of a lung cancer patient who may have a lymph node metastasis; and
determining that the lymph node tissue is a lymph node metastasis of the lung cancer when the acquired mRNA expression level of stratifin is overexpressed.

2. The method of claim 1, wherein the mRNA expression level of stratifin is acquired by nucleic acid amplification method.

3. The method of claim 2, wherein the nucleic acid amplification method is selected from the group consisting of a polymerase-chain-reaction assay, a strand displacement reaction assay, a ligase-chain-reaction assay or a transcriptional amplification method.

4. The method of claim 2, wherein the mRNA expression level of stratifin is a value based on the cycle number of nucleic acid amplification reaction by the nucleic acid amplification method.

5. The method of claim 4, wherein the determining that the lymph node tissue is a lymph node metastasis of the lung cancer comprises:
comparing the cycle number of nucleic acid amplification reaction with a predetermined threshold value; and
determining that the lung cancer has spread to the lymph node tissue when the cycle number is smaller than the predetermined threshold value.

6. The method of claim 5, wherein the determining that the lymph node tissue is a lymph node metastasis of the lung cancer comprises:
determining that the lung cancer has not spread to the lymph node tissue when the cycle number is higher than the predetermined threshold value.

7. The method of claim 2, wherein the mRNA expression level of stratifin is a quantitative value of mRNA of stratifin which is calculated by the nucleic acid amplification method.

8. The method of claim 7, wherein the determining that the lymph node tissue is a lymph node metastasis of the lung cancer further comprises:
comparing the calculated quantitative value of mRNA of stratifin with a predetermined threshold value, and
determining that the lung cancer has spread to the lymph node tissue when the quantitative value is higher than the predetermined threshold value.

9. The method of claim 8, wherein the determining that the lymph node tissue is a lymph node metastasis of the lung cancer further comprises:
determining that the lung cancer has not spread to the lymph node tissue when the quantitative value is smaller than the predetermined threshold value.

10. The method of any one of claims 1 to 9, wherein the lymph node tissue is a tissue containing a lymph node belonging to a lung.

11. The method of claim 10, wherein the lymph node belonging to the lung is any one of mediastinal lymph nodes such as upper longitudinal lymph nodes, paratracheal lymph nodes, pretracheal lymph nodes, anterior mediastinal lymph nodes, retrotracheal lymph nodes, tracheobronchial lymph nodes, subaortic lymph nodes, paraaortic lymph nodes, tracheal-bifurcation lymph nodes, juxtaesophageal lymph nodes and pulmonary ligament lymph nodes; hilar lymph nodes such as primary peribronchial lymph nodes, lobar bronchi lymph nodes and lobar peribronchial lymph nodes; segmental peribronchial lymph nodes, and subsegmental bronchus lymph nodes.

12. The method of any one of claims 1 to 11, wherein the measurement sample prepared using the lymph node tissue is a sample obtained by homogenizing a tissue which may have a metastasis in lung cancer in the pretreatment liquid.

13. The method of claim 12, wherein a pH of the pretreatment liquid is from 2.5 to 5.0.

14. An apparatus for determining lymph node metastasis in lung cancer, comprising:
a measurement unit which acquires mRNA expression level of stratifin contained in a measurement sample prepared by using a lymph node tissue which may have a metastasis in lung cancer;
a determination unit which determines that lung cancer has spread to the lymph node tissue when the mRNA expression of stratifin obtained by the measurement unit is overexpressed; and
an output unit which outputs the determination result obtained by the determination unit.

15. A computer program product, comprising:
a computer readable medium; and
instructions, on the computer readable medium, adapted to enable a general purpose computer to perform operations, comprising:
receiving mRNA expression level of stratifin contained in a measurement sample prepared by using a lymph node tissue which may have a metastasis in lung cancer;
determining that lung cancer has spread to the lymph node tissue when the mRNA expression of stratifin obtained in the receiving step is overexpressed; and
outputting the determination result obtained by the determining step.
